(19) Europäisches Patentamt
European Patent Office
Office européen des brevets

(11) Publication number: **0 046 694**
**A1**

(12) # EUROPEAN PATENT APPLICATION

(21) Application number: 81303902.1

(22) Date of filing: 26.08.81

(51) Int. Cl.³: **C 07 D 333/38**
C 07 D 333/36, C 07 D 409/12
C 07 D 409/06

(30) Priority: 27.08.80 US 181923

(43) Date of publication of application: 03.03.82 Bulletin 82/9

(84) Designated Contracting States: CH DE GB LI

(71) Applicant: EASTMAN KODAK COMPANY
343 State Street
Rochester, New York 14650(US)

(72) Inventor: Krutak, James John
P.O. Box 511
Kingsport Tennessee(US)

(72) Inventor: Maleski, Robert Joseph
P.O. Box 511
Kingsport Tennessee(US)

(74) Representative: Selby-Lowndes, Guy Francis Charles et al,
KODAK LIMITED P.O. Box 114 190 High Holborn
London WC1V 7EA(GB)

(54) **Preparation of thiophenes.**

(57) Process for the preparation of thiophenes having the structure

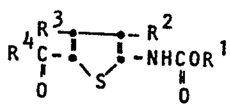

wherein $R^1$, $R^2$, $R^3$ and $R^4$ can be various substituents, by reacting an enamine containing a tertiary amine with a ketone. The thiophenes can be converted to the corresponding 2-amino thiophenes from which azo dyes can be prepared.

EP 0 046 694 A1

PREPARATION OF THIOPHENES

This invention relates to the preparation of certain thiophenes which are useful in the preparation of azo dyes.

This invention is a process for the preparation of thiophenes having the formula

(I) 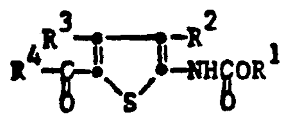

which comprises reacting an enamine having the formula

(II) 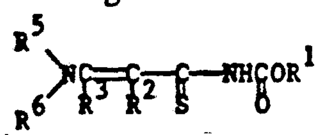

with a ketone having the formula $R^4\text{-}\overset{\text{O}}{\overset{\|}{\text{C}}}\text{CH}_2\text{-X}$ (III) wherein

$R^1$ is an alkyl, cycloalkyl or aryl radical;

$R^2$ is hydrogen, an alkyl, cycloalkyl or aryl radical, or an acyl group (i.e. a residue of an acid), such as alkoxycarbonyl, carboxyl, cyano, carbamoyl, N-alkylcarbamoyl, N,N-dialkylcarbamoyl, N-arylcarbamoyl, alkanoyl, aroyl, piperidinocarbonyl, morpholinocarbonyl, sulfamoyl, N-alkylsulfamoyl, N,N-dialkylsulfamoyl, N-arylsulfamoyl, alkylsulfonyl, arylsulfonyl, piperidinosulfonyl, morpholinosulfonyl, alkylsulfinyl, arylsulfinyl, piperidinosulfinyl or morpholinosulfinyl;

$R^3$ is hydrogen or an alkyl, cycloalkyl or aryl radical;

$R^4$ is an alkyl, cycloalkyl, aryl or a heterocyclic aryl radical containing 5 or 6 ring members;

$R^5$ and $R^6$ are alkyl radicals or together $R^5$ and $R^6$ are tetramethylene, pentamethylene or 3-oxapentamethylene; and

X is chloro or bromo.

The process can be carried out in an inert, organic solvent at a temperature in the range of 15 to 200°C., preferably 20 to 80°C., to give thiophenes (I) in good to excellent yields. Thiophenes (I) can be converted to the corresponding 2-aminothiophenes by using relatively mild conditions which, under certain conditions, do not affect other sensitive groups present on the thiophene compound.

Rajappa et al, Indian J.Chem., 12 (1974) 1-3, disclose that a good yield of thiophene (V) was obtained by the following reaction:

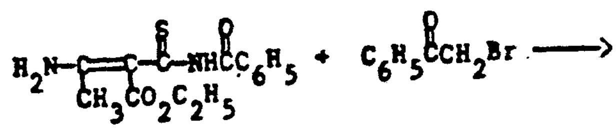

(IV)

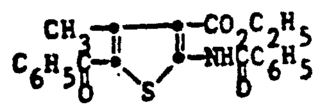

(V)

In attempts to prepare thiophenes (I) by the reaction disclosed by Rajappa et al using an enamine containing a primary amino group, i.e., enamine (II) wherein both $R^5$ and $R^6$ are hydrogen, the predominant product was found to be pyrimidines rather than the desired thiophenes. Thus, in preparing thiophenes (I) containing the $-NHCOR^1$ group rather than a $-NHCR^1$ group, it is essential that the enamine contain a tertiary amine as is present in enamines (II).

The alkyl groups represented by $R^1$, $R^2$, $R^3$ and $R^4$ and the alkyl moieties of the other groups represented by $R^2$ can contain up to 18 carbon atoms, preferably up to 4 carbon atoms (referred to herein as "lower"). The alkyl groups can be substituted with various substituents which do not affect the course of the process, i.e., those that do not react with either (II) or (III). Examples of such substituents include hydroxy, lower alkoxy, cyano, nitro, succinimido, glutarimido, phthalimido, cyclohexyl, fluorenyl, aryl, fluorosulfonyl and groups having the formula $-NH-Y-R^7$, $-Y-R^7$, $-OCOO-R^7$, $-CONR^8R^9$ and $-SO_2NR^8R^9$ wherein $R^8$ is hydrogen, alkyl, cycloalkyl, or aryl and $R^9$ is hydrogen or alkyl, Y is -CO-, -COO-, or $-SO_2-$ and $R^7$ is unsubstituted or substituted alkyl, cycloalkyl, or aryl or when Y is -CO-, $R^7$ also can be amino, alkylamino, dialkylamino, arylamino, or furyl. The alkyl groups represented by $R^7$ preferably are unsubstituted lower alkyl or lower alkyl substituted, for example, with halogen, aryl, cyano, lower alkylsulfonyl, hydroxy, lower alkylthio, lower alkanoyloxy, etc.

The carbocyclic aryl groups represented by $R^1$, $R^2$, $R^3$ and $R^4$ can contain up to 18 carbon atoms and can be unsubstituted or substituted, for example, with alkyl or the substituents which can be present on the alkyl groups represented by $R^1$, $R^2$, $R^3$ and $R^4$. Examples of such aryl groups include unsubstituted and substituted phenyl and naphthyl.

The heterocyclic aryl groups which $R^4$ can represent contain 5 or 6 ring members, are stabilized by resonance and can be unsubstituted or substituted

with alkyl or with one or more of the substituents which can be present on the above-described alkyl radicals. Examples of the heterocyclic aryl groups include pyridyl, quinolyl, oxazolyl, benzoxazolyl, thiazolyl, benzothiazolyl, benzimidazolyl, oxadiazolyl, the thiadiazolyls, thienyl, pyrimidinyl and pyrazinyl.

The process is especially useful in preparing thiophenes of formula (I) from enamines wherein $R^2$ is an alkoxycarbonyl group having the formula $-COO-R^{10}$ wherein $R^{10}$ is a carboxyl-protecting group, i.e., a group which in conjunction with -COO- forms an ester from which a carboxyl group can be generated. Examples of such groups are set forth in Chapter 5 of Halsam, Protective Groups in Organic Chemistry, Plenum Press (1973). The preferred groups represented by $R^{10}$ are lower alkyl such as 1,1-dimethylethyl; 2-cyanoethyl; 2,2,2-trichloroethyl; arylmethyl such as benzyl, p-methoxybenzyl, 2,4,6-trimethylbenzyl, and pentamethylbenzyl; benzhydryl;2-arylsulfonylethyl such as 2-phenylsulfonylethyl; 2-aroylethyl such as 2-benzoylethyl; and phthalimidomethyl including substituted phthalimidomethyl such as 4-sulfamoyl- and 4-lower alkylsulfamoylphthalimidomethyl.

Examples of inert organic solvents which can be employed in the process are ethers such as diethyl ether, tetrahydrofuran and p-dioxane, alcohols such as methanol, isopropanol, and butanol, ether-alcohols such as 2-methoxyethanol and 2-(2-methoxyethoxy)-ethanol, hydrocarbons such as hexane and toluene and pyridine compounds such as pyridine and the picolines.

The process is particularly suitable for the preparation of thiophenes (I) by reacting enamines

(II) with a ketone having the formula $R^4-\overset{O}{\overset{\|}{C}}CH_2Br$ wherein:

$R^1$ is lower alkyl, 2,2,2-trichloroethyl, 1,1-dimethyl-2,2,2-trichloroethyl or phenyl;

$R^2$ is hydrogen, lower alkyl, phenyl, cyano, benzoyl or a group having the formula $-COO-R^{10}$ wherein $R^{10}$ is a carboxyl-protecting group;

$R^3$ is hydrogen, lower alkyl or phenyl;

$R^4$ is lower alkyl, cyclohexyl or phenyl substituted with hydroxy, sulfamoyl, lower alkylsulfamoyl, sulfo, fluorosulfonyl, lower alkoxysulfonyl, carboxyl, lower alkylsulfonamido, arylsulfonamido, nitro, cyano, chloro, lower alkoxy or lower alkylsulfonyl; and

$R^5$ and $R^6$ each is lower alkyl.

EXAMPLE 1

A mixture of 25 g (.061 m) of ethyl 2-[[(2,2,2-trichloroethoxycarbonyl)amino]thioxomethyl]-3-(diethylamino)-propenoate, 17.15 g (.061 m) of m-fluorosulfonylphenacyl bromide, and 125 ml isopropyl alcohol was heated to reflux for five minutes. The reactants dissolved at reflux and shortly thereafter the product precipitated. The mixture was cooled and the solid collected by filtration. The solid was washed with isopropyl alcohol and then water to give 27.6 g (85%) of product, ethyl 5-[3-(fluorosulfonyl)benzoyl]-2-[[2,2,2-trichloroethoxycarbonyl]-amino]-3-thiophenecarboxylate, melting point 108-110°C. Ir 2.85, 5.72, 5.91, 6.09, 6.70, 7.10, 8.10, 8.42; NMR ($CDCl_3$) 10.7 (s,1); 8.40 (t,1); 8.2 (m,2); 7.8 (t,1); 7.70 (s,1); 4.90 (s,2); 4.37 (q,2); 1.36 (t,3). Anal: Calcd for $C_{17}H_{13}Cl_3FNO_7S_2$: C: 38.32; H: 2.46; N: 2.63; Found: C: 38.27; H: 2.60; N: 2.69.

This compound can be converted to the corresponding 2-amino compound by treating an isopropyl alcohol solution of it with zinc dust at reflux temperature.

EXAMPLE 2

A mixture of 130 g of ethyl 2-[[(phenoxycarbonyl)amino]thioxomethyl]-3-(diethyl-amino)-propenoate (0.37 mole), 104 g of m-fluorosulfonylphenacyl bromide (0.37 mole), and 350 ml of isopropanol was heated to reflux and held one hour. During this time, the reactants dissolved and the product precipitated. The mixture was cooled to 20-25°C, filtered, washed with isopropanol and then water to yield 133 g of white solid, ethyl 5-[3-(fluorosulfonyl)benzoyl]-2-[(phenoxycarbonyl)-amino]-3-thiophenecarboxylate, (75% yield), melting point 161-163°C. Ir: 2.88, 5.72, 5.98, 6.18, 7.12, 8.10, 8.40 μ; NMR ($CDCl_3$) δ 10.75 (s,1,N-H); 8.4 (m,1); 8.2 (m,2); 7.8 (m,1); 7.7 (s,1, thiophene-proton); 4.4 (q,2); 1.4 (t,3).

EXAMPLE 3

The following example illustrates the preparation of ethyl 5-[3-(fluorosulfonyl)benzoyl]-2-[(ethoxycarbonyl)amino]-3-thiophene carboxylate starting with ethyl propiolate and diethylamine.

A solution of 1.96 g (.02 mole) ethyl propiolate in 20 ml p-dioxane was stirred at 0-5°C. and treated with 1.46 g diethylamine. The ice bath cooling was removed and an exothermic reaction occurred. The temperature increased to 35°C. but was checked there by cooling with an ice bath. After stirring 2 hours at 5-25°C., 2.62 g (.02 mole) ethoxycarbonyl isothiocyanate was added. After 3-1/2 hours an additional .3 g ethoxycarbonyl isothiocyanate (3) was added. One and one-half hours later

a solution of 5.62 g (.02 mole) m-fluorosulfonylphenacyl bromide in 15 ml p-dioxane was added at 8°C. An exothermic reaction occurred. The temperature increased unchecked to 33°C. The reaction was stirred for several hours and filtered. The solid product that was collected was washed with isopropanol and then water and dried in air to give 4.12 g thiophene product, m.p. 131-132°C. From the filtrate was obtained 2.89 g additional product. Total yield 7.01 g (82%). The structure was assigned on the basis of infrared, NMR, and elemental analysis. Anal. Calcd. for $C_{17}H_{16}FNO_7S_2$: C, 47.55; H, 3.76; N, 3.26. Found C, 47.55; H, 3.85; N, 3.20.

EXAMPLE 4

A mixture of 15 g (0.41 mole) 1,1-dimethylethyl-3-(diethyl)amino-2-[[(phenoxycarbonyl)amino]-thioxomethyl]-2-butenoate and 8.19 g (.041 mole) phenacyl bromide in 100 ml isopropyl alcohol was refluxed for one hour and stirred one hour at room temperature. The solid product was filtered and washed in succession with isopropanol, water, and isopropanol. After drying in air, the product, 1,1-dimethylethyl-5-benzoyl-4-methyl-2-[(phenoxycarbonyl)amino]-3-thiophenecarboxylate, weighed 16.5 g (92%) and had a melting point of 183°C. The structure was established by infrared, NMR, and elemental analyses. Anal. Calcd. for $C_{24}H_{23}NO_5S$: C, 65.88; H, 5.30; N, 3.20. Found: C, 65.72; H, 5.33; N, 3.32.

If Example 2 is repeated using 1,1-dimethylethyl 3-amino-2-[[(phenoxycarbonyl)amino]thioxomethyl]-2-butenoate as the enamine reactant, the predominant product obtained is 1,1-dimethylethyl

4-[(benzoylmethyl)thio]-1,2-dihydro-6-methyl-2-oxo-5-pyrimidinecarboxylate having the structure

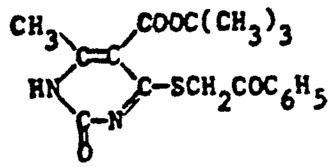

The compounds set forth in the following examples illustrate other thiophenes of formula (I) which can be prepared according to this process.

| Example | $R^1$ | $R^2$ | $R^3$ | $R^4$ | Melting Point, °C. |
|---|---|---|---|---|---|
| 5 | $-C_2H_5$ | $-\overset{O}{\overset{\Vert}{C}}OCH_3$ | H | 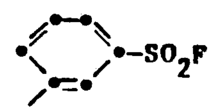 | 153–155 |
| 6 | $-C_2H_5$ | $-\overset{O}{\overset{\Vert}{C}}OC_2H_5$ | H | $-CH_3$ | 95–96.5 |
| 7 | $-CH_2CCl_3$ | $-\overset{O}{\overset{\Vert}{C}}OC_2H_5$ | H | $C_6H_5$ | 123–125 |
| 8 | $-CH_2CCl_3$ | $-\overset{O}{\overset{\Vert}{C}}OC_2H_5$ | H | 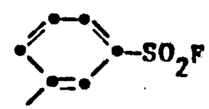 | 108–110 |
| 9 | $-CH_2CH_2Cl$ | $-\overset{O}{\overset{\Vert}{C}}OC_2H_5$ | H | 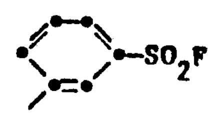 | 136–137 |
| 10 | $-C_6H_5$ | $-\overset{O}{\overset{\Vert}{C}}OC_2H_5$ | H | $-\overset{O}{\overset{\Vert}{C}}CH_3$ | 131–133 |
| 11 | $-C_6H_5$ | $-\overset{O}{\overset{\Vert}{C}}OC_2H_5$ | H | 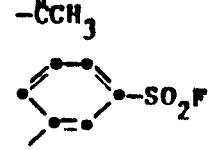 | 160–162 |
| 12 | $-C_6H_5$ | $-CN$ | H | $-C_6H_5$ | 204–211 |
| 13 | $-C_2H_5$ | $-CN$ | H | $-C_6H_5$ | 164–166 |
| 14 | $-C_2H_5$ | $-\overset{O}{\overset{\Vert}{C}}C_6H_5$ | H | $-C_6H_5$ | 148–150.5 |

| | | | | | |
|---|---|---|---|---|---|
| 15 | $-C_2H_5$ | 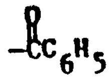 | H | 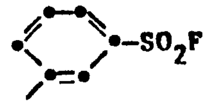 | 197-198.5 |
| 16 | 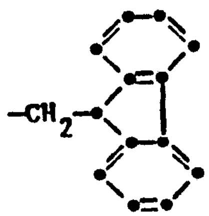 | 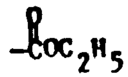 | H | 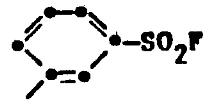 | 158-160 |
| 17 | $-C_6H_5$ | 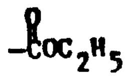 | H | 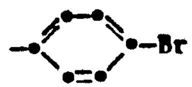 | 172-174 |
| 18 | $-C_6H_5$ | 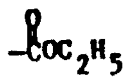 | H | 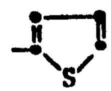 | 163-164.5 |
| 19 | $-C_6H_5$ | 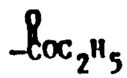 | H | 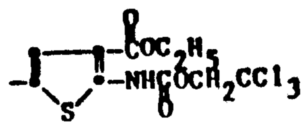 | 180-181 |
| 20 | $-CH_2CCl_3$ | 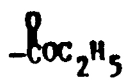 | H | 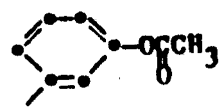 | 142-143.5 |
| 21 | $-C_6H_5$ | 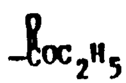 | H | 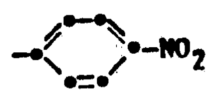 | 140.5-141.5 |
| 22 | $-C_2H_5$ | 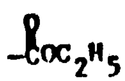 | H | 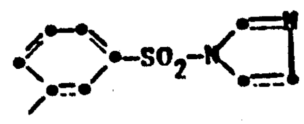 | 159-161 |

| | | | | | |
|---|---|---|---|---|---|
| 23 | $-C_6H_5$ | $-\overset{O}{\overset{\|}{C}}OC_2H_5$ | H | 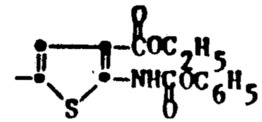 | 221–222 |
| 24 | $-\underset{CH_3}{\overset{CH_3}{C}}-CCl_3$ | $-\overset{O}{\overset{\|}{C}}OC_2H_5$ | H | 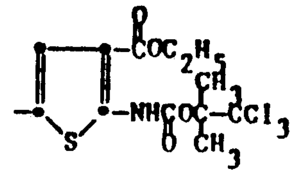 | 263–264 |
| 25 | $-C_6H_5$ | $-\overset{O}{\overset{\|}{C}}OC_2H_5$ | H | 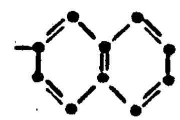 | 121–123 |
| 26 | $-CH_2CCl_3$ | $-\overset{O}{\overset{\|}{C}}OC(CH_3)_3$ | H | 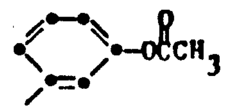 | 155–156 |
| 27 | $-CH_2CCl_3$ | $-\overset{O}{\overset{\|}{C}}OCH_2CH_2CN$ | H | 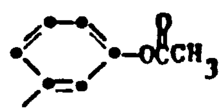 | 136–137 |
| 28 | $-C_6H_5$ | $-\overset{O}{\overset{\|}{C}}OC_2H_5$ | H | $-CH_2Br$ | 111–113 |
| 29 | $-CH_2CCl_3$ | $-\overset{O}{\overset{\|}{C}}OCH_2CH_2CN$ | H | 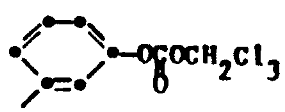 | 146–148 |
| 30 | $-C_6H_5$ | $-\overset{O}{\overset{\|}{C}}OCH_2CH_2CN$ | H | 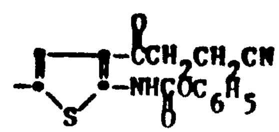 | 220–222 |

| | | | | | |
|---|---|---|---|---|---|
| 31 | $-CH_2CCl_3$ | $-\overset{O}{\overset{\Vert}{C}}OCH_2CF_3$ | H | 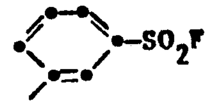 | 131.5–133.5 |
| 32 | $-CH_2CCl_3$ | $-\overset{O}{\overset{\Vert}{C}}OCH_2CH_2CN$ | H | 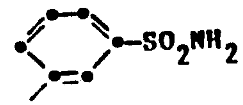 | 177–178 |
| 33 | $-CH_2CCl_3$ | $-\overset{O}{\overset{\Vert}{C}}OCH_2CH_2CN$ | H | 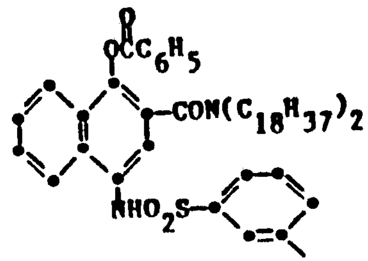 | – |
| 34 | $-CH_2CCl_3$ | $-\overset{O}{\overset{\Vert}{C}}OCH_2CH_2CN$ | H | 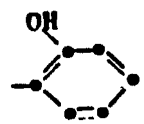 | 176–178 |
| 35 | $-CH_2CCl_3$ | 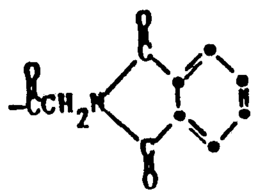 | H | $-C_6H_5$ | 188–190 |
| 36 | $-CH_2CCl_3$ | 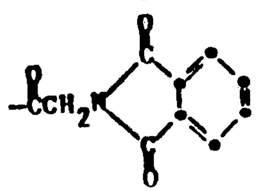 | H | 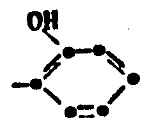 | 174–176 |

0046694



Claims:

1. A process for the preparation of thiophenes having the formula

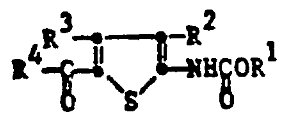

characterized by reacting an enamine having the formula

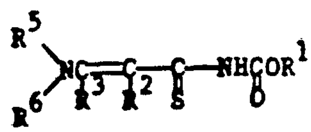

with a ketone having the formula $R^4-\overset{O}{\overset{\|}{C}}CH_2-X$ wherein

$R^1$ is an alkyl, cycloalkyl or aryl;

$R^2$ is hydrogen, an alkyl, cycloalkyl or aryl radical, alkoxycarbonyl, carboxyl, cyano, carbamoyl, N-alkylcarbamoyl, N,N-dialkylcarbamoyl, N-arylcarbamoyl, alkanoyl, aroyl, piperidinocarbonyl, morpholinocarbonyl, sulfamoyl, N-alkylsulfamoyl, N,N-dialkylsulfamoyl, N-arylsulfamoyl, alkylsulfonyl, arylsulfonyl, piperidinosulfonyl, morpholinosulfonyl, alkylsulfinyl, arylsulfinyl, piperidinosulfinyl or morpholinosulfinyl;

$R^3$ is hydrogen or an alkyl, cycloalkyl or aryl radical;

$R^4$ is an alkyl, cycloalkyl, aryl or a heterocyclic aryl radical containing 5 or 6 ring members;

$R^5$ and $R^6$ are alkyl radicals or together $R^5$ and $R^6$ are tetramethylene, pentamethylene or 3-oxapentamethylene; and

X is chloro or bromo.

2. The process according to Claim 1 wherein $R^2$ is an alkoxycarbonyl group having the formula $-COO-R^{10}$ wherein $R^{10}$ is a carboxyl-protecting group.

3. The process according to Claim 1 wherein $R^2$ is an alkoxycarbonyl group having the formula $-COO-R^{10}$ wherein $R^{10}$ is lower alkyl, 2-cyanoethyl, arylmethyl, benzhydryl, 2-arylsulfonylethyl, 2-aroylethyl, phthalimidomethyl and phthalimidomethyl substituted at the 4-position with sulfamoyl or lower alkylsulfamoyl.

4. The process according to Claim 1 wherein the reaction is carried out in an inert organic solvent at a temperature of 15 to 200°C.

5. A process for the preparation of a thiophene having the formula

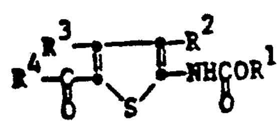

which comprises reacting an enamine having the formula

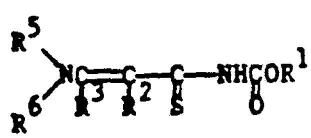

with a ketone having the formula $R^4-\overset{O}{\overset{\|}{C}}CH_2-Br$ wherein

$R^1$ is lower alkyl, 2,2,2-trichloroethyl, 1,1-dimethyl-2,2,2-trichloroethyl or phenyl;

$R^2$ is hydrogen, lower alkyl, phenyl, cyano, benzoyl or a group having the formula $-COO-R^{10}$ wherein $R^{10}$ is a carboxyl-protecting group;

$R^3$ is hydrogen, lower alkyl or phenyl;

$R^4$ is lower alkyl, cyclohexyl or phenyl substituted with hydroxy, sulfamoyl, lower alkylsulfamoyl, sulfo, fluorosulfonyl, lower alkoxysulfonyl, carboxyl, lower alkylsulfonamido, arylsulfonamido, nitro, cyano, chloro, lower alkoxy or lower alkylsulfonyl; and

$R^5$ and $R^6$ each is lower alkyl.

European Patent Office

**EUROPEAN SEARCH REPORT**

0046694

Application number

EP 81 30 3902

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| D | CHEMICAL ABSTRACTS, vol. 81, no. 11, 16th September 1974, page 451, no. 63420e Columbus, Ohio, U.S.A. S. RAJAPPA et al.: "Synthesis of thiophenes. III. Further variations in the substitution pattern" & INDIAN J. CHEM. 1974, 12(1), 1-3 * Abstract * ---- | 1-3 | C 07 D 333/38 333/36 409/12 409/06 |
| | | | TECHNICAL FIELDS SEARCHED (Int. Cl.3) |
| | | | C 07 D 333/38 333/36 409/12 409/06 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| The Hague | 14-10-1981 | CHOULY |

EPO Form 1503.1 06.78